# EUROPEAN PATENT APPLICATION

(11) **EP 4 574 398 A1**
(43) Date of publication of application: **25.06.2025**
(21) Application number: 23854742.6
(22) Date of filing: 10.07.2023
(51) Int. Cl.: B29C 64/314, B29C 64/124

(54) **CATIONICALLY POLYMERIZABLE RESIN COMPOSITION FOR 3D STEREOLITHOGRAPHY AND METHOD FOR PRODUCING 3D STEREOLITHOGRAPHY PRODUCT**

(30) Priority: 18.08.2022 JP 2022130688
(71) Applicant: Tokuyama Dental Corporation, Tokyo 110-0016 (JP)
(72) Inventor: KASUYA, Reina, Tokyo 110-0016 (JP); SAKATA, Eibu, Tokyo 110-0016 (JP); NAKASHIMA, Kei, Tokyo 110-0016 (JP)
(74) Representative: HGF
(86) International application number: PCT/JP2023/025432
(87) International publication number: WO 2024/038705

(57) **Abstract**

Provided is a cationically polymerizable resin composition for three-dimensional photofabrication that contains a cationically polymerizable monomer (A), a photocationic polymerization initiator (B) containing a photoacid generator (b1), a photosensitizer (b2), and an electron-donating compound (b3) that is at least one type of thiophene compound selected from the group consisting of monothiophene compounds having a π-conjugated substituent as a substituent, dithiophene compounds, and trithiophene compounds, and a basic compound (C). The content of the electron-donating compound (b3) is 0.001-10 parts by mass and the content of the basic compound (C) is 0.0001-5.0 parts by mass per 100 parts by mass of the cationically polymerizable monomer (A). Also provided is a method for producing a three-dimensional photofabricated product using this cationically polymerizable resin composition for three-dimensional photofabrication.

## Description

### TECHNICAL FIELD

The present invention relates to a cationically polymerizable resin composition for three-dimensional photofabrication and a method for producing a three-dimensional photofabricated product using the composition.

### BACKGROUND ART

A technique of producing a three-dimensional photofabricated product by curing a photocurable resin composition by irradiation with light is known as three-dimensional photofabrication. Among the three-dimensional photofabrication processes, there are widely known a vat photopolymerization process where a photocurable resin composition placed in a vat is irradiated with light to achieve the fabrication and a material jetting process where a photocurable resin composition is ejected by an ink-jet printer and the ejected photocurable resin composition is concurrently irradiated with light to achieve the fabrication.

In the dental field, a dental model, a surgical guide, an individual tray, a dental aligner model, and a dental restoration (e.g., a denture or a crown prosthesis) need to be produced highly accurately so as to have a unique shape in accordance with the situation in the oral cavity of each individual patient, and photofabrication based on computer-aided design (CAD) data designed on the basis of digital data obtained by oral scanning or the like can easily produce a three-dimensional photofabricated product that reproduces the designed shape with high accuracy. Among them, the vat photopolymerization process is often employed in the dental field from the viewpoints of durability of the resulting three-dimensional photofabricated product, accuracy during fabrication, or a fabrication rate, etc.

As used herein, a vat photopolymerization process means a method for producing a three-dimensional object having a shape corresponding to a shape of a three-dimensional object by (1) digitizing and ordinating the three-dimensional object in a height direction of the three-dimensional object and generating two-dimensional shape data indicating a cross-sectional shape of the three-dimensional object at each ordinated height, based on three-dimensional shape data indicating the shape of the three-dimensional object; (2) irradiating a predetermined position of a liquid photocurable resin composition held in a vat with activating light being ultraviolet light or visible light to selectively cure the liquid photocurable resin composition present in the position to thereby form a fabricated layer having the cross-sectional shape and a predetermined thickness (also referred to as "stacking pitch"); and (3) repeating procedures of moving the thus-formed fabricated layer or a height at which the light is irradiated upward or downward by the predetermined thickness (stacking pitch) and then forming a fabricated layer having a shape corresponding to a two-dimensional shape at the moved-height according to an order of the ordinating to thereby sequentially stack newly-formed fabricated layers.

In the dental field, a "lifting" three-dimensional photofabrication device is becoming more and more popular among such vat photopolymerization processes. The "lifting" means a method in which a base, called a platform, is raised by lifting it up in accordance with a photofabrication rate, so that a fabricated layer is stacked while the fabricated layer is moved upward. The "lifting" three-dimensional photofabrication device can use a shallow resin tray as a vat, enabling fabrication with a small amount of resin.

By the way, a radical polymerizable resin composition using a photoinitiator which generates radicals by active light emitted from a light source built into a three-dimensional photofabrication device together with a radical polymerizable monomer such as a (meth)acrylic compound, as described in Patent Document 1, is the mainstream of a resin composition for three-dimensional photofabrication.

Such a radical polymerizable resin composition is known to have high polymerization shrinkage during curing. Generally, a three-dimensional photofabrication device incorporates a setting for fabrication by irradiating a larger area than an original CAD data with light in order to reduce a difference between a resulting three-dimensional photofabricated product and the CAD data caused by polymerization shrinkage when the radical polymerizable resin composition is polymerized. However, volume shrinkage is not uniform depending on a shape of the three-dimensional photofabricated product, which causes a decrease in fabrication accuracy. In addition, a three-dimensional photofabricated product fabricated using a radical polymerizable resin composition is subject to residual stress inside due to volume shrinkage to cause a gradual dimensional change or distortion, which is particularly problematic in manufacture of a dental model, etc. where high accuracy must be maintained over a long period of time.

On the other hand, a cationically polymerizable monomer, especially one that provides a cured product by ring-opening polymerization, is characterized by low polymerization shrinkage. For this reason, a dental curable composition using a cationically polymerizable monomer has also been developed. For example, Patent Document 2 describes a "dental composition useful for polymerization in an oral environment, the composition including a) a cationically polymerizable epoxy resin, b) a hydroxyl-containing substance, c) an aryl iodonium salt, and d) a visible light sensitizer which is an α-dicarbonyl compound having an absorption coefficient less than about 1000 (L·mol⁻¹·cm⁻¹) in a visible region".

In addition, as a resin composition for optical three-dimensional modeling (resin composition for three-dimensional photofabrication) that can provide a cured product with low shrinkage during curing and good load deflection and tensile elongation, a resin composition for optical three-dimensional modeling containing (A) a cationically polymerizable organic compound having one or two or more epoxide structures in its molecule, (B) a polyether having hydroxy groups at both ends with an average molecular weight of 500 to 10000, and (C) an energy ray-sensitive cationic polymerization initiator composed of a double salt that is an onium salt which releases a Lewis acid upon irradiation with an energy ray or a derivative thereof (see Patent Document 3).

### Citation List

### Patent Document

Patent Document 1: Japanese Unexamined Patent Application, Publication No. 2020-158417
Patent Document 2: Japanese Unexamined Patent Application (Translation of PCT Application), Publication No. H10-508067
Patent Document 3: Japanese Unexamined Patent Application, Publication No. 2003-073457

### DISCLOSURE OF THE INVENTION

### Problems to be Solved by the Invention

It is believed that use of cationically polymerizable resin compositions as described in Patent Documents 2 and 3 for three-dimensional photofabrication can suppress a dimensional change and distortion caused by polymerization shrinkage. Therefore, the present inventors tried to apply the above-mentioned cationically polymerizable resin compositions to fabrication of a dental restoration using a lifting three-dimensional photofabrication device which has been widely employed in the dental field. As a result, it was confirmed that there were problems as described below when the compositions were applied as they were. That is, in the case of the cationically polymerizable resin composition described in Patent Document 2, it was confirmed that there were problems in that dimensions of the resulting fabricated product were different from the data and that sufficient fabrication accuracy was not able to be achieved due to thin and small protrusions called a "burr" that was formed on a stack interface of fabricated layers. In addition, it was found that the cationically polymerizable resin composition described in Patent Document 3 not only showed an increase in viscosity when stored for a long period of time, but also sometimes caused the resulting fabricated product to fall off in the middle of a fabrication process when a commercially available lifting three-dimensional photofabrication device is used probably due to an insufficient curing property during fabrication of fabricated layers. Moreover, it was also found that for both the cationically polymerizable resin compositions described in Patent Documents 2 and 3, if the resin composition in a vat (resin tray) was used repeatedly multiple times without being replaced upon photofabrication, fabrication accuracy further deteriorated at the early stages of the photofabrication such as the second or third photofabrication, and thus the fabricated product fell off in some cases.

Therefore, an object of the present invention is to provide a cationically polymerizable resin composition for three-dimensional photofabrication that has good storage stability, that enables fabrication with high accuracy upon fabrication using a lifting three-dimensional photofabrication device, and that is resistant to degradation in fabrication accuracy even after being repeatedly used for fabrication.

### Means for Solving the Problems

Specific means for solving the above-mentioned object include the following embodiments.
<1> A cationically polymerizable resin composition for three-dimensional photofabrication, the composition including: a cationically polymerizable monomer (A);
   a photocationic polymerization initiator (B) including a photo acid generator (b1), a photosensitizer (b2), and an electron-donating compound (b3) the electron-donating compound being at least one thiophene compound selected from the group consisting of a monothiophene compound having a π-conjugated substituent as a substituent, a dithiophene compound, and a trithiophene compound; and
   a basic compound (C),
   the electron-donating compound (b3) being contained in an amount of 0.001 to 10 parts by mass and the basic compound (C) being contained in an amount of 0.0001 to 5.0 parts by mass relative to 100 parts by mass of the cationically polymerizable monomer (A).
<2> The cationically polymerizable resin composition for three-dimensional photofabrication according to <1>, in which the electron-donating compound (b3) is at least one selected from a dithiophene compound or a trithiophene compound and the basic compound (C) is a hindered amine compound.
<3> The cationically polymerizable resin composition for three-dimensional photofabrication according to <1> or <2>, in which the photosensitizer (b2) is at least one selected from the group consisting of a thioxanthone compound, an α-diketone compound, and an anthracene compound.
<4> The cationically polymerizable resin composition for three-dimensional photofabrication according to any one of <1> to <3>, further including at least one selected from an activating light absorbent (D) or a phenolic antioxidant (E).
<5> A method for producing a three-dimensional photofabricated product, the method including:
   producing a stack having a shape corresponding to a shape of a three-dimensional object by digitizing and ordinating the three-dimensional object in a height direction of the three-dimensional object and generating two-dimensional shape data indicating a cross-sectional shape of the three-dimensional object at each ordinated height, based on three-dimensional shape data indicating the shape of the three-dimensional object, and sequentially forming and stacking fabricated layers each having a shape corresponding to a two-dimensional shape at each height based on the two-dimensional shape data, according to an order of the ordinating, using a vat photopolymerization device in which a predetermined position of a liquid cationically polymerizable resin composition held in a vat is irradiated with activating light being ultraviolet light or visible light to selectively cure the liquid cationically polymerizable resin composition present in the position;
   the liquid cationically polymerizable resin composition being the cationically polymerizable resin composition for three-dimensional photofabrication according to any one of <1> to <4>.
<6> The method for producing a three-dimensional photofabricated product according to <5>, in which a dental restoration is produced as the three-dimensional photofabricated product.

### Effects of the Invention

A cationically polymerizable resin composition for three-dimensional photofabrication of the present invention has excellent features, that is, has good storage stability, enables fabrication with high accuracy upon fabrication using a lifting three-dimensional photofabrication device, and is resistant to degradation in fabrication accuracy even after being repeatedly used for fabrication. A method for producing a three-dimensional fabricated product of the present invention also makes it possible to faithfully reproduce three-dimensional shape data and produce a product of interest with high accuracy.

### PREFERRED MODE FOR CARRYING OUT THE INVENTION

Specific embodiments to which the present invention is applied will be described in detail. It should be noted that in this specification, the expression "x to y" using numerical values x and y is intended to mean "x or more and y or less" unless otherwise specified. When only the numerical value y is described with its unit in such a notation, the unit shall also be applied to the numerical value x.

### <Cationically polymerizable resin composition for three-dimensional photofabrication>

Generally, a minor amount of a basic compound is added to a cationically polymerizable resin composition to thereby trap the generated cationic species in order to improve stability of the cationically polymerizable resin composition. The present inventors believe that in a three-dimensional photofabrication using a cationically polymerizable resin composition by a vat photopolymerization process, a cationic species generated during light irradiation relatively stably remains in and diffuses into an uncured resin composition and thus polymerization proceeds even in an environment where light is blocked, which causes a decrease in fabrication accuracy and generation of a "burr". Therefore, the present inventors conceived and studied the idea of adding a basic compound to the cationically polymerizable resin composition in order to neutralize the cationic species. However, as a result, a polymerization activity was decreased and sufficient curing was not able to be achieved during light irradiation in three-dimensional photofabrication. This is probably because the basic compound inhibited a polymerization initiation reaction of the cationically polymerizable resin composition.

Therefore, in order to obtain a photocationic polymerization initiator with a high activity during light irradiation even in the presence of the basic compound, an effect of adding an electron-donating compound to a photocationic polymerization initiator composed of a photo acid generator and a photosensitizer was examined. As a result, the present inventors have found that a desired effect is not able to be obtained when an electron-donating compound such as an aromatic amine, an alkyl aryl polyether, an anthracene compound, etc., which is known to improve an activity of the cationically polymerizable resin composition, is added and that the above problem can be solved by adding a specific thiophene compound. Thus, the present invention has been completed.

In other words, a cationically polymerizable resin composition for three-dimensional photofabrication according to the present embodiment (hereinafter simply referred to as "cationically polymerizable resin composition according to the present embodiment") includes a cationically polymerizable monomer (A); a photocationic polymerization initiator (B) including a photo acid generator (b1), a photosensitizer (b2), and an electron-donating compound (b3) which is at least one thiophene compound selected from the group consisting of a monothiophene compound having a π-conjugated substituent as a substituent, a dithiophene compound, and a trithiophene compound; and a basic compound (C), the electron-donating compound (b3) being contained in an amount of 0.001 to 10 parts by mass and the basic compound (C) being contained in an amount of 0.0001 to 5.0 parts by mass relative to 100 parts by mass of the cationically polymerizable monomer (A).

A cationically polymerizable resin composition according to the present embodiment has excellent features, that is, has good storage stability, enables fabrication with high accuracy upon fabrication using a lifting three-dimensional photofabrication device, and is resistant to degradation in fabrication accuracy even after being repeatedly used for fabrication.

The reason for this effect is not necessarily clear, but it is assumed that a high activity enhancement effect could be achieved since a thiophene compound used in the cationically polymerizable resin composition according to the present embodiment is not only highly electron-donating due to a π-conjugation effect, but also generates an acid via a cation radical produced by electron transfer (in other words, also functions as a photoacid generator).

A cationically polymerizable resin composition according to the present embodiment has excellent features as described above. Therefore, the cationically polymerizable resin composition according to the present embodiment can be suitably used as a material for fabricating various products in three-dimensional photofabrication, especially for fabricating a dental photofabricated product. For example, a dental model needs to have a unique shape with high accuracy in accordance with oral conditions of each patient, and is often stored for a long period of time. Hence, it is suitable to perform photofabrication using the cationically polymerizable resin composition according to the present embodiment based on CAD data designed on the basis of digital data obtained by intraoral scanning or the like.

Hereinafter, components contained in a cationically polymerizable resin composition according to the present embodiment will be described in detail. Regarding each of the components described below, one or two or more of the materials listed for each component may be used alone or in any combination.

### [Cationically polymerizable monomer (A)]

A cationically polymerizable monomer (A) serving as a polymerizable component is not particularly limited as long as it is a compound that polymerizes by the action of an acid generated by decomposition of a photoacid generator, and those used as a cationically polymerizable monomer in conventional photocation curable compositions can be used without restriction. Examples of a cationically polymerizable monomer include an epoxy compound, an oxetane compound, a cyclic ether compound, a vinylether compound, a bicyclic orthoester compound, a cyclic acetal compound, a bicyclic acetal compound, or a cyclic carbonate compound. Among them, at least one selected from an epoxy compound or an oxetane compound is preferred from the viewpoints of its easy availability, small volume shrinkage, and fast polymerization reaction.

Examples of an epoxy compound that can be suitably used include a compound having one epoxy functional group in its molecule such as 1,2-epoxypropane, methylglyzidyl ether, cyclohexene oxide, exo-2,3-epoxynorbornene, 4-vinyl-1-cyclohexene-1,2-epoxide, limonene oxide, α-pinene oxide, styrene oxide, (2,3-epoxypropyl)benzene, phenyl glycidyl ether; a compound having two epoxy functional groups in its molecule such as 1,3-butadiene dioxide, ethylene glycol diglycidyl ether, diethylene glycol diglycidyl ether, triethylene glycol diglycidyl ether, 1,6-hexanediol diglycidyl ether, diglycidyl glutarate, 4-vinyl-1-cyclohexene dipoxide, limonene dipoxide, methyl bis[2-(7-oxabicyclo[4.1.0]hept-3-yl)ethyl]phenyl silane; a compound having three or more epoxy functional groups in its molecule such as glycerol triglycidyl ether, trimethylolpropane triglycidyl ether, pentaerythritol triglycidyl ether, pentaerythritol tetraglycidyl ether, dipentaerythritol hexaglycidyl ether; a compound having cyclic siloxane and silsesquioxane structures that has an epoxy functional group (e.g., compounds represented by formulae below); etc. In the following formulae, g to i represent integers falling within numerical ranges described in the formulae.

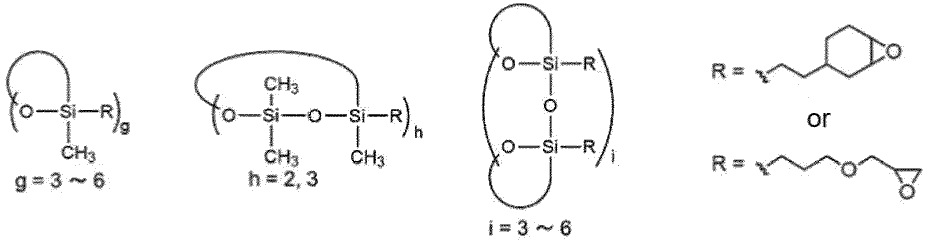

Among these epoxy compounds, a compound having two or more epoxy functional groups per molecule is preferred from the viewpoint of a physical property of the resulting cured product.

Also, examples of an oxetane compound that can be suitably used include a compound having one oxetane ring in its molecule such as trimethylene oxide, 3-methyl-3-oxetanylmethanol, 3-ethyl-3-oxetanylmethanol, 3-ethyl-3-phenoxymethyloxetane, 3,3-diethyloxetane, 3-ethyl-3-(2-ethylhexyloxy)oxetane; a compound having two or more oxetane rings in its molecule such as 1,4-bis(3-ethyl-3-oxetanylmethyloxy)benzene, 4,4'-bis(3-ethyl-3-oxetanylmethyloxy)biphenyl, 4,4'-bis(3-ethyl-3-oxetanylmethyloxymethyl)biphenyl, ethylene glycol bis(3-ethyl-3-oxetanylmethyl)ether, diethylene glycol bis(3-ethyl-3-oxetanylmethyl ether) (e.g., compounds represented by formulae below); etc. In the following formulae, j to v represent integers falling within numerical ranges described in the formulae.

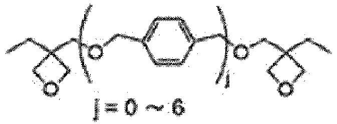

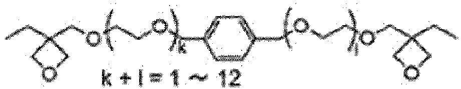

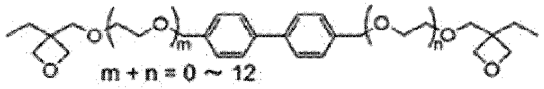

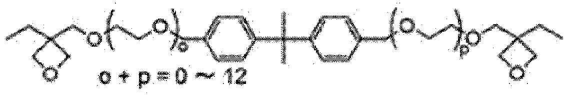

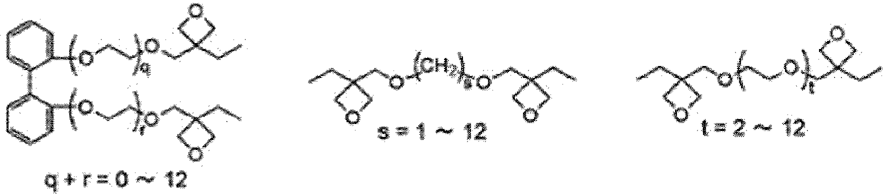

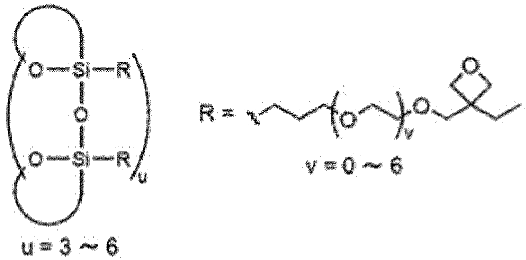

Among these oxetane compounds, a compound having two or more oxetane rings per molecule is preferred from the viewpoint of a physical property of the resulting cured product.

These cationically polymerizable monomers may be used alone or two or more thereof may be used in combination. In particular, a mixture of "A" moles of an oxetane compound having an average of "a" oxetane functional groups per molecule and "B" moles of an epoxy compound having an average of "b" epoxy functional groups per molecule, which has been adjusted so that a ratio (a x A):(b x B) falls within a range of 90:10 to 10:90, is suitable from the viewpoints of a fast curing rate and resistance to polymerization inhibition by moisture.

### [Photocationic polymerization initiator (B)]

A photocationic polymerization initiator (B) includes a photoacid generator (b1), a photosensitizer (b2), and an electron-donating compound (b3).

### (Photoacid generator (b1))

A photoacid generator (b1) is not particularly limited as long as it generates a strong acid by a reaction upon light irradiation, and those used as a photoacid generator in conventional photocation curable compositions can be used without restriction. Examples of a photoacid generator include an onium salt compound such as an iodonium salt compound, a sulfonium salt compound, a bismuthonium salt compound, or a pyridinium salt compound. Among them, an iodonium salt compound is preferred from the viewpoints of its easy availability and high polymerization activity.

Examples of a cation or a cationic moiety in an onium salt compound include diphenyliodonium, bis(p-chlorophenyl)iodonium, ditolyliodonium, bis(p-tert-butylphenyl)iodonium, p-isopropylphenyl-p-methylphenyl iodonium, bis(m-nitrophenyl)iodonium, p-tert-butylphenylphenyl iodonium, p-metoxyphenylphenyliodonium, bis(p-methoxyphenyl)iodonium, p-octyloxyphenylphenyliodonium, p-phenoxyphenylphenyliodonium, bis(p-dodecylphenyl)iodonium, triphenylsulfonium, tritolylsulfonium, p-tert-butylphenyl diphenylsulfonium,diphenyl-4-phenylthiophenylsulfonium, diphenyl-2,4,6-trimethylphenylsulfonium, tetraphenylbismuthonium, triphenyl-2,4,6-trimethylphenylbismuthonium, 1-methylpyridinium, 1-methyl2-chloropyridinium, etc.

Examples of an anion or an anionic moiety of an onium salt compound include tetrakispentafluorophenylborate, tetra (nonafluoro-tert-butoxy) aluminate, hexafluorophosphate, hexafluoroantimonate, hexafluoroarsenate, tetrafluoroborate, trifluoromethanesulfonate, perchlorate, etc.

A content of a photoacid generator (b1) is not particularly limited as long as it is an amount that can initiate polymerization upon light irradiation. However, if the content is too high, a dark reaction is likely to progress after the light irradiation, which may cause deterioration in storage stability and a decrease in fabrication accuracy after repeated use of the resin in fabrication. Whereas, if the content is too small, a three-dimensional photofabricated product may not be fabricated due to low strength of the resulting cured product after light irradiation. Therefore, in order to achieve both an appropriate polymerization progress rate and various physical properties (e.g., weather resistance or hardness) of the resulting cured product, the content of a photoacid generator (b1) is usually 0.01 to 20 parts by mass, preferably 0.05 to 10 parts by mass, and more preferably 0.1 to 3 parts by mass relative to 100 parts by mass of a cationically polymerizable monomer (A).

### (Photosensitizer (b2))

A photosensitizer (b2) is not particularly limited as long as it is a compound that absorbs activating light emitted from a three-dimensional photofabrication device to be used and facilitates decomposition of a photoacid generator (b1). Examples of a photosensitizer include a cyanine dye, a merocyanine dye, a thiazine dye, a xanthene dye, an azine dye, a squarylium dye, a pyrylium salt dye, an acridine dye, a benzoflavin dye, a fused polycyclic aromatic compound (e.g., anthracene, perylene, etc.), a phenothiazine compound, a diaryl ketone compound, an α-diketone compound, a coumarin compound, a xanthone compound, a thioxanthone compound, etc. Among them, an anthracene compound, a thioxanthone compound, an α-diketone compound, or a coumarin compound is preferred.

Examples of a particularly suitable photosensitizer include an anthracene compound represented by Formula (4) below; a thioxanthone compound represented by Formula (5) below; or at least one α-diketone compound selected from the group consisting of camphorquinone, benzyl, phenylpropanedione, diacetyl, acetyl benzoyl, 2,3-pentadione, 2,3-octadione, 4,4'-dimethoxybenzyl, 4,4'-oxybenzyl, 9,10-phenanthrenequinone, and acenaphthenequinone.

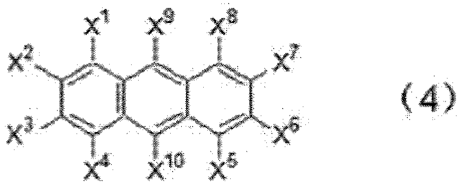

In Formula (4) above, X¹ to X¹⁰ each independently represents a hydrogen atom, an alkyl group having 1 to 20 carbon atoms, a halogen atom, an alkoxy group having 1 to 20 carbon atoms, or an acyl group. Among the compounds represented by Formula (4) above, 2-ethyl-9,10-dimethoxyanthracene, 9,10-dibutoxyanthracene, or 9,10-dimethylanthracene is preferred.

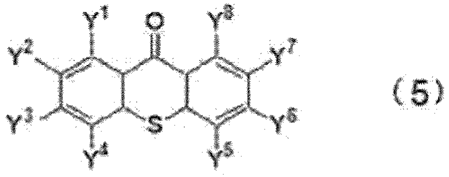

In Formula (5) above, Y¹ to Y⁸ each independently represents a hydrogen atom, an alkyl group having 1 to 20 carbon atoms, a halogen atom, an alkoxy group having 1 to 20 carbon atoms, or an acyl group. Among the compounds represented by the Formula (5) above, thioxanthone, 2-isopropylthioxanthone, 2,4-dimethylthioxanthone, or 2,4-diethylthioxanthone is preferred.

An α-diketone compound is more preferably camphorquinone or benzyl, and further preferably benzyl.

A content of a photosensitizer (b2) varies depending on a type of other components to be combined or a polymerizable monomer. However, if the content is too high, activating light absorbed by the photosensitizer is excessive, and the activating light does not sufficiently reach an area in a fabricated layer far from the side irradiated with the activating light (due to attenuation of the activating light), which may result in formation of an area that is insufficiently cured. Whereas, if the content is too small, the resulting cured product after light irradiation has low strength since sufficient energy cannot be supplied to the photoacid generator, and a three-dimensional photofabricated product may not be fabricated. Therefore, the content of a photosensitizer (b2) is usually 0.001 to 10 parts by mass, preferably 0.005 to 5 parts by mass, and further preferably 0.01 to 1.0 parts by mass relative to 100 parts by mass of a cationically polymerizable monomer (A).

### (Electron-donating compound (b3))

A cationically polymerizable resin composition according to the present embodiment needs to contain, as an electron-donating compound (b3), at least one thiophene compound selected from the group consisting of a monothiophene compound having a π-conjugated substituent as a substituent, a dithiophene compound, and a trithiophene compound (hereinafter also referred to as "specific thiophene compound") in an amount of 0.001 to 10 parts by mass relative to 100 parts by mass of a cationically polymerizable monomer (A). A specific thiophene compound is believed to exert a high activity enhancement effect because of not only its high electron-donating property due to an π-conjugation effect but also its function as a photoacid generator as described above.

If a specific thiophene compound is not included or its content is below the above-mentioned lower limit (0.001 parts by mass), sufficient curing is not achieved during light irradiation due to an insufficient activity enhancement effect, and a three-dimensional fabricated product is more likely to fall off in the middle of photofabrication. Conversely, if the content exceeds the above-mentioned upper limit (10 parts by mass), excessive acid is generated during light irradiation, which causes a decrease in fabrication accuracy. From the viewpoint of the effect, a content of a specific thiophene compound is preferably 0.005 to 5 parts by mass and more preferably 0.01 to 1.5 parts by mass relative to 100 parts by mass of a cationically polymerizable monomer (A).

A specific thiophene compound is preferably at least one thiophene compound selected from the group consisting of a monothiophene compound represented by Formula (1) below, a dithiophene compound represented by Formula (2) below, and a trithiophene compound represented by Formula (3) below.

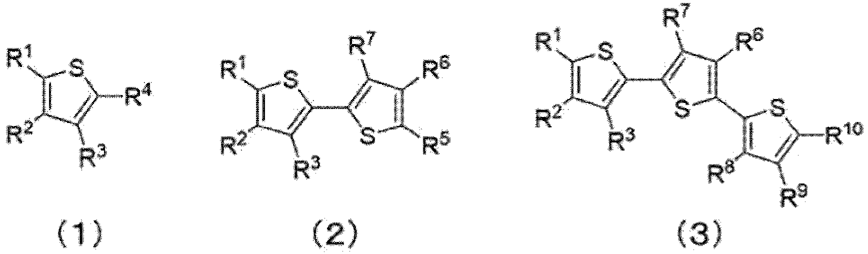

In Formulae (1) to (3) above, R¹ to R¹⁰ each independently represents a hydrogen atom, a halogen atom, an alkyl group having 1 to 12 carbon atoms, an alkenyl group having 2 to 12 carbon atoms, an alkynyl group having 2 to 12 carbon atoms, an alkoxy group having 1 to 12 carbon atoms, an alkylthio group having 1 to 12 carbon atoms, an aryl group, or a heteroaryl group. However, at least one of R¹ to R⁴ in Formula (1) above represents a π-conjugated substituent. Examples of a π-conjugated substituent include an aryl group such as a phenyl group or a fluorenyl group; a heteroaryl group such as a furyl group, a pyrrolyl group, or a thienyl group; etc. A π-conjugated substituent may be substituted with an alkyl group having 1 to 12 carbon atoms, a halogen atom, etc., and may be further substituted with another π-conjugated substituent.

Among the specific thiophene compounds, a dithiophene compound or trithiophene compound in which at least one of carbon atoms at position 2 has no substituent is preferably used from the viewpoints of a more extended π-conjugation and a structure that easily releases an acid. In other words, a dithiophene compound in which at least one of R¹ or R⁵ in Formula (2) above is a hydrogen atom or a trithiophene compound in which at least one of R¹ or R¹⁰ in Formula (3) above is a hydrogen atom is preferably used.

Specific examples of a specific thiophene compound include 2-phenylthiophene, 2,5-diphenylthiophene, 2,3,4,5-tetraphenylthiophene, 2,2'-(9,9-dioctyl-9H-fluorene-2,7-diyl)bisthiophene, 2,2'-bithiophene, 5-hexyl-2,2'-bithiophene, 5-octyl-2,2'-bithiophene, 5-bromo-2,2'-bithiophene, 3,3'-dihexyl-2,2'-bithiophene, 4,4'-dihexyl-2,2'-bithiophene, 5,5'-dihexyl-2,2'-bithiophene, 2,2':5',2"-terthiophene, 5,5"-dibromo-2,2':5',2"-terthiophene, etc.

Note that, a compound other than a specific thiophene compound can be incorporated as an electron-donating compound (b3) as long as it does not interfere with the effect of the invention, but it is preferred that the electron-donating compound (b3) consists of the specific thiophene compound.

### [Basic compound (C)]

A cationically polymerizable resin composition according to the present embodiment needs to contain 0.0001 to 5.0 parts by mass of a basic compound (C) relative to 100 parts by mass of a cationically polymerizable monomer (A). A basic compound (C) is believed to have a function of trapping a cationic species generated by activating light from a three-dimensional photofabrication device. By trapping a cationic species, progress of polymerization is suppressed in an environment where light is blocked and cationic polymerization in an undesired area by the action of diffused acid is also suppressed during photofabrication, which improves storage stability and enables high-accuracy fabrication without generating a "burr" even after three-dimensional photofabrication is repeatedly performed multiple times.

If a content of a basic compound is too high, an acid generated during irradiation with activating light is not used for a polymerization reaction and thus the polymerization reaction is inhibited, so three-dimensional photofabrication does not proceed and a desired three-dimensional photofabricated product cannot be obtained. If the content of a basic compound is too low, generation of a burr cannot be suppressed and thus fabrication accuracy is decreased. For such a reason, the content of a basic compound is preferably 0.0005 to 0.5 parts by mass and more preferably 0.0005 to 0.05 parts by mass relative to 100 parts by mass of a cationically polymerizable monomer (A).

A basic compound (C) may be an inorganic base or an organic base as long as it is soluble in a cationically polymerizable monomer (A) and can trap a generated cationic species. Examples of an inorganic base include an alkali metal hydride such as sodium hydride, etc.; an alkaline earth metal hydride such as calcium hydride, etc.; an alkali metal hydroxide such as sodium hydroxide, lithium hydroxide, cesium hydroxide, etc.; an alkaline earth metal hydroxide such as calcium hydroxide, etc.; an alkali metal carbonates such as lithium carbonate, potassium carbonate, etc.; an alkaline earth metal carbonate such as calcium carbonate, etc.; cesium fluoride; etc. Also, examples of an organic base include an alkali metal salt or ammonium salt of an organic acid such as sodium dodecyl sulfate, ammonium dodecyl sulfate, sodium p-toluenesulfonate, etc.; a metal alkoxide such as potassium-t-butoxide, sodium ethoxide, etc.; an aromatic amine such as ethyl p-dimethylaminobenzoate, p-dimethylaminotoluene, etc.; an aliphatic amine such as triethylamine, propylamine, diisopropylethylamine, etc.; a hindered amine such as bis(2,2,6,6-tetramethyl-4-piperidyl) sebacate, bis(1,2,2,6,6-pentamethyl-4-piperidyl) sebacate, N,N'-bis(2,2,6,6-tetramethylpiperidyl-4-yl)hexane-1,6-diamine, N,N'-bis(2,2,6,6-tetramethylpiperidin-4-yl) isophthalamide, tetrakis(1,2,2,6,6-pentamethyl-4-piperidyl)butane-1,2,3,4-tetracarboxylate, a mixed esterified product of 1,2,3,4-butanetetracarboxylic acid with 1,2,2,6,6-pentamethyl-4-piperidinol and 3,9-bis(2-hydroxy-1,1-dimethylethyl)-2,4,8,10-tetraoxaspiro[5.5]undecane; a polymer compound having a basic functional group in a polymer chain such as polyvinylpyrrolidone, polyacrylonitrile; etc. Among them, a hindered amine is preferred from the viewpoint of its high stability after trapping a cationic species.

### [Activating light absorbent (D)]

In order to prevent excessive transmission of activating light emitted from a three-dimensional photofabrication device and, in turn, a decrease of fabrication accuracy of the resulting three-dimensional photofabricated product, a cationically polymerizable resin composition according to the present embodiment may contain an activating light absorbent (D). An activating light absorbent (D) is not particularly limited as long as it is a compound that absorbs activating light emitted from a three-dimensional photofabrication device and may be, for example, a benzophenone compound, a benzotriazole compound, etc., and, specifically, 2,2',4,4'-tetrahydroxybenzophenone, 2-(2-hydroxy-3-tert-butyl-5-methylphenyl)-5-chlorobenzotriazole, 2-(3,5-tert-amyl-2-hydroxyphenyl)benzotriazole, etc.

When a cationically polymerizable resin composition according to the present embodiment contains an activating light absorbent (D), a content thereof is preferably 0.04 to 3.0 parts by mass relative to 100 parts by mass of a cationically polymerizable monomer (A). If the content of an activating light absorbent (D) is less than the above-mentioned lower limit (0.04 parts by mass), activating light is excessively transmitted, and thus fabrication accuracy tends to be low. If the content is higher than the above-mentioned upper limit (3.0 parts by mass), activating light does not sufficiently transmit and thus a fabricated product may not be fabricated. From the viewpoint of the effect, the content of an activating light absorbent (D) is more preferably 0.06 to 2.0 parts by mass and further preferably 0.08 to 1.4 parts by mass relative to 100 parts by mass of a cationically polymerizable monomer (A).

### [Other components]

A cationically polymerizable resin composition according to the present embodiment may contain other components of which type or content does not impair the effect of the present invention, if necessary, in addition to the above-mentioned components.

### (Phenolic antioxidant (E))

A cationically polymerizable resin composition according to the present embodiment may contain a phenolic antioxidant (E) as an additive for suppressing decomposition of a photoacid generator. Examples of a phenolic antioxidant (E) include 4-methoxyphenol, hydroquinone, 2,6-di-t-butylphenol, dibutylhydroxytoluene, 2,4-di-t-butylphenol, 2-t-butyl-4,6-dimethylphenol, etc.

When a cationically polymerizable resin composition according to the present embodiment contains a phenolic antioxidant (E), a content thereof is preferably 0.001 to 10 parts by mass and more preferably 0.01 to 1.0 part by mass relative to 100 parts by mass of a cationically polymerizable monomer (A).

### (Inorganic particles)

A cationically polymerizable resin composition according to the present embodiment may contain inorganic particles in order to increase mechanical strength of the resulting three-dimensional fabricated product.

A material for inorganic particles is not particularly limited, and for example, any of those for use as a filler for a tooth restoration material may be used. Specific examples thereof include a single metal; a metal oxide or a metal composite oxide; a metal fluoride or a metal salt such as a carbonate, a sulfate, a silicate, a hydroxide, a chloride, a sulfite, or a phosphate; a mixture of these metal salts; etc. Among them, a metal oxide such as amorphous silica, quartz, alumina, titania, zirconia, barium oxide, yttrium oxide, lanthanum oxide, or ytterbium oxide; a silica-based composite oxide such as silica-zirconia, silica-titania, silica-titania-barium oxide, or silica-titania-zirconia; glass such as borosilicate glass, aluminosilicate glass, or fluoroaluminosilicate glass; a metal fluoride such as barium fluoride, strontium fluoride, yttrium fluoride, lanthanum fluoride, or ytterbium fluoride; an inorganic carbonate such as calcium carbonate, magnesium carbonate, strontium carbonate, or barium carbonate; a metal sulfate such as magnesium sulfate or barium sulfate; or the like is preferred. In the case of production of a dental restoration, particles of a silica-based composite oxide such as silica-zirconia, silica-titania, silica-titania-barium oxide, silica-titania-zirconia, or the like are preferably used because of their strong X-ray contrast property and silica-zirconia particles are more preferably used from the viewpoint of wear resistance of the resulting cured product.

A particle diameter of these inorganic particles is not particularly limited, and inorganic particles having an average particle diameter of 0.01 to 100 µm (preferably 0.01 to 10 µm), which are commonly used as a tooth restoration material, may be appropriately used depending on the purposes. Furthermore, inorganic particles may be incorporated as the so-called organic-inorganic composite filler. Incidentally, the above-mentioned inorganic particles are desirably treated with a surface treating agent typified by a silane coupling agent in order to improve compatibility with a polymerizable monomer and increase mechanical strength and water resistance. The surface treatment may be performed according to any known method and, for example, methyltrimethoxysilane, methyltriethoxysilane, methyltrichlorosilane, dimethyldichlorosilane, trimethylchlorosilane, vinyltrichlorosilane, vinyltriethoxysilane, vinyltris(β-methoxyethoxy)silane, γ-methacryloyloxypropyltrimethoxysilane, methacryloxyoctyl-8-trimethoxysilane, γ-chloropropyltrimethoxysilane, γ-glycidoxypropylmethoxysilane, hexamethyldisilazane, or the like is suitably used as a silane coupling agent.

When a cationically polymerizable resin composition according to the present embodiment contains inorganic particles, if a content thereof is too high, a viscosity of the cationically polymerizable resin composition becomes too high. For such a reason, the content of inorganic particles is preferably 300 parts by mass or less and more preferably 200 parts by mass or less relative to 100 parts by mass of a cationically polymerizable monomer (A).

### (Other additives)

When a cationically polymerizable resin composition according to the present embodiment is used as a dental composition, the cationically polymerizable resin composition may contain, in addition to the above-mentioned components, other additives known to be components for dental curable compositions. Such additives include, for example, a dye, an antistatic agent, a pigment, a fragrance, an organic solvent, or a thickening agent.

### <Method for producing three-dimensional photofabricated product>

A method for producing a three-dimensional photofabricated product according to the present embodiment (hereinafter also simply referred to as "production method according to the present embodiment") is a method including a shaping step in which a stack having a shape corresponding to a shape of a three-dimensional object is produced by digitizing and ordinating the three-dimensional object in a height direction of the three-dimensional object and generating two-dimensional shape data indicating a cross-sectional shape of the three-dimensional object at each ordinated height, based on three-dimensional shape data indicating the shape of the three-dimensional object, and sequentially forming and stacking fabricated layers each having a shape corresponding to a two-dimensional shape at each height based on the two-dimensional shape data, according to an order of the ordinating, using a vat photopolymerization device in which a predetermined position of a liquid cationically polymerizable resin composition held in a vat is irradiated with activating light being ultraviolet light or visible light to selectively cure the liquid cationically polymerizable resin composition present in the position; the method using the above-mentioned cationically polymerizable resin composition according to the present embodiment as the liquid cationically polymerizable resin composition.

In a production method according to the present embodiment, the shaping step preferably includes:
a first step of irradiating a predetermined position of a liquid cationically polymerizable resin composition held in a vat with activating light and curing the composition, based on two-dimensional shape data at a first ordinate height in an ordinating order, to form a fabricated layer having a shape corresponding to the two-dimensional shape data, the fabricated layer being used as a prebonding layer;
a second step of moving the prebonding layer upward to supply the liquid cationically polymerizable resin composition directly below the prebonding layer in the vat;
a third step of irradiating a predetermined position of the liquid cationically polymerizable resin composition supplied directly below the prebonding layer with activating light and
curing the composition, based on two-dimensional shape data at a height of a next ordinate in the ordinating order following the immediately preceding step, and thereby forming a new fabricated layer having a shape corresponding to the two-dimensional shape data, and concurrently bonding the new fabricated layer to the prebonding layer, to obtain a stack having the new fabricated layer as a new prebonding layer; and
a fourth step of moving the stack upward to supply the liquid cationically polymerizable resin composition directly below the new prebonding layer in the vat;
a cycle consisting of the third step and the fourth step is repeated using the new prebonding layer as the prebonding layer in the third step, and in the last third step, a new fabricated layer is formed based on two-dimensional shape data at a height of the last ordinate in the ordinating order to obtain a stack.

A vat photofabrication process including such a shaping step can be suitably performed using a commercially available vat photopolymerization device, the so-called lifting 3D printer.

In a production method according to the present embodiment, after the shaping step, the resulting stack is preferably washed with an organic solvent (such a step is also referred to as a "washing step") and then secondarily cured by additional irradiation with activating light, heat treatment, or both (such a step is also referred to as a "secondary curing step").

Examples of an organic solvent to be used in a washing step include an alcohol solvent such as ethanol, methanol, or isopropyl alcohol; a ketone solvent such as acetone or methyl ethyl ketone; an ether solvent such as diethyl ether, diisopropyl ether, tripropylene glycol monomethyl ether, or tetrahydrofuran; an amide solvent such as N-methylpyrrolidone or dimethylacetamide; a halogen solvent such as methylene chloride or chloroform; or the like. Among them, an alcohol solvent or an ether solvent is preferred from the viewpoint of high washing efficiency and an alcohol solvent is more preferred from the viewpoint of low environmental load.

When additional irradiation with activating light is performed in a secondary curing step, an irradiation wavelength is not particularly limited as long as it is a wavelength that a photopolymerization initiator remaining in a stack absorbs to generate a radical. An irradiation intensity in the additional irradiation with activating light is preferably 5 mW/cm² or more, more preferably 10 mW/cm² or more, and further more preferably 30 mW/cm² or more in order for a photopolymerization initiator remaining in a stack to generate a sufficient amount of radicals. An irradiation time is not particularly limited, and is preferably 1 minute or longer, more preferably 3 minutes or longer, and further preferably 5 minutes or longer. Note that, an excessively high irradiation intensity during the additional irradiation with activating light leads to excessive heating of a three-dimensional photofabricated product, which may cause a crack. Therefore, the irradiation intensity is preferably 10,000 mW/cm² or less.

### EXAMPLES

Hereinafter, the present invention will be described more specifically by way of examples, but the present invention is not limited to these examples.

Compounds used in Examples and Comparative Examples and abbreviations thereof are shown below.

### (1) Cationically polymerizable monomer (A)

· KR-470: Compound represented by the following formula (manufactured by Shin-Etsu Chemical Co., Ltd.)
· OXT-121: Compound represented by the following formula (manufactured by TOAGOSEI CO., LTD.)

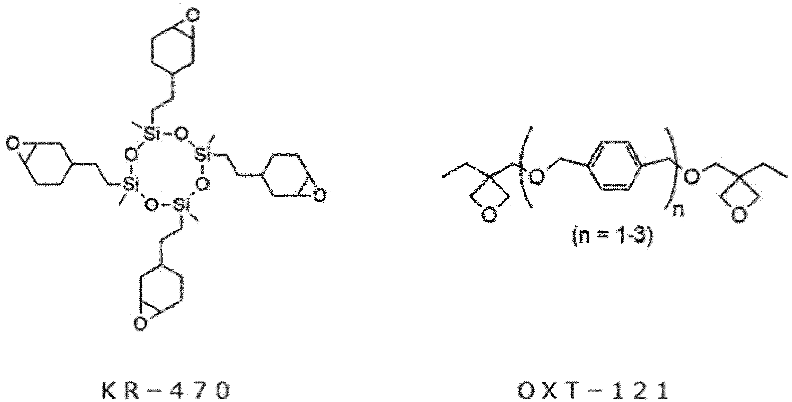

### (2) Photoacid generator (b1)

· DPIB: Iodonium salt with tetrakis-pentafluorophenylborate as a counter anion, represented by the following formula (manufactured by Tokyo Chemical Industry Co., Ltd)

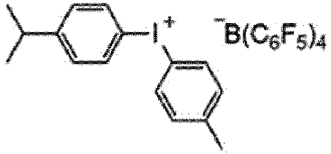

### (3) Photosensitizer (b2)

· IPTO: 2-isopropylthioxanthone (manufactured by Tokyo Chemical Industry Co., Ltd)
· BZ: Benzyl (dibenzoyl) (manufactured by Tokyo Chemical Industry Co., Ltd)
· CQ: Camphorquinone (manufactured by Tokyo Chemical Industry Co., Ltd)
· DMEAn: 2-ethyl-9,10-dimethoxyanthracene (manufactured by FUJIFILM Wako Pure Chemical Corporation)

### (4) Electron-donating compound (b3)

### (4-1) Specific thiophene compound

· DThFlu: 2,2'-(9,9-dioctyl-9H-fluorene-2,7-diyl)bisthiophene (manufactured by Sigma-Aldrich)
· PTh: 2-Phenylthiophene (manufactured by Tokyo Chemical Industry Co., Ltd)
· O-2T: 5-octyl-2,2'-bithiophene (manufactured by Tokyo Chemical Industry Co., Ltd)
· 3T: 2,2':5',2"-terthiophene (manufactured by Tokyo Chemical Industry Co., Ltd)

### (4-2) Compound other than specific thiophene compound

· DMBE: Ethyl 4-(N,N'-dimethylamino)benzoate (manufactured by Tokyo Chemical Industry Co., Ltd)

### (5) Basic compound (C)

. Tinuvin 770: Bis(2,2,6,6-tetramethylpiperidin-4-yl) sebacate (manufactured by Tokyo Chemical Industry Co., Ltd)
· B5642: Bis(2,2,6,6-tetramethyl-4-piperidyl-1-oxyl) sebacate (manufactured by Tokyo Chemical Industry Co., Ltd)
· B6154: N,N'-bis(2,2,6,6-tetramethyl-4-piperidinyl)isophthalamide (manufactured by Tokyo Chemical Industry Co., Ltd)
· LA-52: Tetrakis(1,2,2,6,6-pentamethyl-4-piperidyl)butane-1,2,3,4-tetracarboxylate (manufactured by ADEKA CORPORATION) · LA-63P: Mix esterified product of 1,2,3,4-butane tetracarboxylic acid with 1,2,2,6,6-pentamethyl-4-piperidinol and 3,9-bis(2-hydroxy-1,1-dimethylethyl)-2,4,8,10-tetraoxaspiro[5.5]undecane (manufactured by ADEKA CORPORATION)

### (6) Activating light absorbent (D)

· SS703: 2-(2-hydroxy-3-tert-butyl-5-methylphenyl)-5-chlorobenzotriazole (manufactured by Tokyo Chemical Industry Co., Ltd)
· SS704: 2-(3,5-tert-amyl-2-hydroxyphenyl) benzotriazole (manufactured by Tokyo Chemical Industry Co., Ltd)
· SS106: 2,2',4,4'-tetrahydroxybenzophenone (manufactured by Tokyo Chemical Industry Co., Ltd)

### (7) Phenolic antioxidant (E)

· HQME: 4-methoxyphenol (manufactured by FUJIFILM Wako Pure Chemical Corporation)
· BHT: Dibutylhydroxytoluene (manufactured by Tokyo Chemical Industry Co., Ltd)

### <Example 1>

### (1) Preparation of cationically polymerizable resin composition

First, 0.6 parts by mass of DPIB serving as an acid generator, 0.3 parts by mass of IPTO serving as a photosensitizer, 0.5 parts by mass of O-2T serving as a thiophene compound, and 0.01 parts by mass of Tinuvin770 serving as a basic compound were added to 100 parts by mass of a cationically polymerizable monomer consisting of 50 parts by mass of KR-470 and 50 parts by mass of OXT-121, and stirred under red light for 12 hours to thereby prepare a cationically polymerizable resin composition.

### (2) Fabrication of three-dimensional photofabricated product (First)

Two hundred grams of the thus-prepared cationically polymerizable resin composition was supplied to a resin tank of a lifting vat photopolymerization device (Form 2, manufactured by Formlabs) to perform a first photofabrication based on Stereolithography data (hereinafter abbreviated as "stl data") of a rectangular prism with a dimension of 60 mm x 70 mm x 10 mm. A fabricated product was able to be obtained without any trouble such as peeling off of a cured product (fabricated layer) suspended from a stage in the middle of photofabrication or inhibition of progress of fabrication caused by a cured product (fabricated layer) remaining in the resin tank. Occurrence of chipping (micro defects) or a burr was not found by visual observation of the resulting fabricated product. Therefore, the resulting fabricated product was evaluated for fabrication accuracy by the following method.

Note that, if evaluation criteria for fabricability were defined as follows based on a status of a trouble during fabrication, the evaluation result for fabricability of Example 1 is determined as "good".

### -Fabricability evaluation criteria-

Good: The fabricated product was able to be fabricated without chipping.
Poor: The fabricated product was not fabricated at all or fell off in the middle of photofabrication.

### (3) Evaluation of fabrication accuracy

Three-dimensional shape data was collected from the fabricated product obtained from the first photofabrication using a 3D scanner (manufactured by KEYENCE CORPORATION), lengths of sides of the fabricated product were compared with those of the stl data, a difference: (Length of side of fabricated product: mm) - (Standard value of side of stl data: mm) was calculated for each side, an average of the differences was calculated, and the thus-calculated average value was evaluated as an average value of fabrication accuracy. The average value of fabrication accuracy was determined as 0.05 mm. By comparing both of the above data, an area that partially protrudes 2.0 mm or more from the stl data was identified as a "burr" and its presence and occurrence were checked, and a surface of the three-dimensional photofabricated product was evaluated as "A" based on the following evaluation criteria.

### -Evaluation criteria of surface of three-dimensional photofabricated product-

A: No burrs were generated.
B: Minute burrs were slightly generated but acceptable.
C: A large number of burrs were generated and large burrs were detached and remained in the resin tank.

### (4) Fabrication of three-dimensional photofabricated product (Second and Third)

After the first (or second) photofabrication, the fabricated product was removed, photofabrication was performed in the same manner as for the first photofabrication using the cationically polymerizable resin composition in the resin tank used in the first (or second) photofabrication as it was, and fabricability and a surface of the resulting three-dimensional photofabricated product were evaluated in the same manner as described above. As a result, for both the second and third fabrication, the fabricability was evaluated as "good" and the surface of the three-dimensional photofabrication product was evaluated as "A" or "B".

### <Examples 2 to 5 and Comparative Examples 1 to 4>

Cationically polymerizable resin compositions were prepared in the same manner as in Example 1, except that amounts of the photosensitizer, the thiophene compound, and the basic compound were changed as described in Table 1, a first to third photofabrication were performed, and fabricability and a surface of the resulting three-dimensional photofabricated product were evaluated for each photofabrication in the same manner as in Example 1. The results are shown in Table 2. Note that, if the fabricability was evaluated as "poor," the subsequent photofabrication was not performed.

**[Table 1]**

| | Photosensitizer (b2) | Electron-donating compound (b3) | Basic compound (C) |
|---|---|---|---|
| | IPTO | Specific thiophene compound: O-2T | Tinuvin770 |
| Example 1 | 0.3 | 0.5 | 0.01 |
| Example 2 | 0.01 | 0.01 | 0.0005 |
| Example 3 | 1 | 1.5 | 0.05 |
| Example 4 | 0.003 | 0.005 | 0.0002 |
| Example 5 | 2 | 3 | 0.2 |
| Comparative Example 1 | 0.3 | - | 0.01 |
| Comparative Example 2 | 0.3 | - | - |
| Comparative Example 3 | 0.3 | 0.5 | - |
| Comparative Example 4 | 0.3 | 0.5 | 8 |

| | | | |
|---|---|---|---|
| * Numerical values in the table represent amounts (part(s) by mass) of components added. * Cationically polymerizable monomer : KR-470 (50 parts by mass), OXT-121 (50 parts by mass) * Photoacid generator : DPIB (0.6 parts by mass) | | | |

**[Table 2]**

| | First | | | Second | | | Third | | |
|---|---|---|---|---|---|---|---|---|---|
| | Fabrica bility | Fabrication accuracy | | Fabrica bility | Fabrication accuracy | | Fabrica bility | Fabrication accuracy | |
| | | Average value | Surface condition | | Average value | Surface condition | | Average value | Surface condition |
| Example 1 | Good | 0.05 | A | Good | 0.05 | A | Good | 0.05 | B |
| Example 2 | Good | 0.06 | B | Good | 0.06 | B | Good | 0.07 | B |
| Example 3 | Good | 0.06 | A | Good | 0.07 | A | Good | 0.07 | B |
| Example 4 | Good | 0.06 | B | Good | 0.07 | B | Good | 0.07 | B |
| Example 5 | Good | 0.07 | A | Good | 0.07 | B | Good | 0.08 | B |
| Comparative Example 1 | Poor | - | - | - | - | - | - | - | - |
| Comparative Example 2 | Poor | - | - | - | - | - | - | - | - |
| Comparative Example 3 | Good | 0.05 | B | Good | 0.06 | C | Poor | - | - |
| Comparative Example 4 | Poor | - | - | - | - | - | - | - | - |

As shown in Examples 1 to 5 in Tables 1 and 2, when the cationically polymerizable resin compositions containing appropriate amounts of the photosensitizers, the specific thiophene compounds, and the basic compounds were used for photofabrication, fabrication proceeded to the end in all three times. On the other hand, when the cationically polymerizable resin compositions that contained no thiophene compounds were used for photofabrication (Comparative Examples 1 and 2), photofabrication did not proceed. When the cationically polymerizable resin composition that did not contain only the basic compound was used for photofabrication (Comparative Example 3), burrs were generated on the surface of the resulting three-dimensional photofabricated product in the first time, indicating a decrease in fabrication accuracy. When the cationically polymerizable resin composition containing an excessive amount of the basic compound was used for photofabrication (Comparative Example 4), the photofabrication did not proceed.

### <Examples 6 to 16 and Comparative Example 5>

Cationically polymerizable resin compositions were prepared in the same manner as in Example 1, except that types and amounts of the photosensitizer, the electron-donating compound, and the basic compound incorporated were changed as described in Table 3, a first to third photofabrication were performed, and fabricability and a surface of the resulting three-dimensional photofabricated product were evaluated for each photofabrication in the same manner as in Example 1. The results are shown in Table 4.

**[Table 3]**

| | Photosensitizer (b2) | Electron-donating compound | | Basic compound (C) |
|---|---|---|---|---|
| | | Specific thiophene compound | Aromatic amine compound | |
| Example 6 | DMEAn (0.3) | O-2T (0.5) | - | Tinuvin770 (0.01) |
| Example 7 | BZ (0.3) | O-2T (0.5) | - | Tinuvin770 (0.01) |
| Example 8 | CQ (0.3) | O-2T (0.5) | - | Tinuvin770 (0.01) |
| Example 9 | DMEAn (0.3), BZ (0.3) | O-2T (0.5) | - | Tinuvin770 (0.01) |
| Example 10 | IPTO (0.3) | DThFlu (1.0) | - | Tinuvin770 (0.01) |
| Example 11 | IPTO (0.3) | PTh (0.3) | - | Tinuvin770 (0.01) |
| Example 12 | IPTO (0.3) | 3T (0.4) | - | Tinuvin770 (0.01) |
| Example 13 | IPTO (0.3) | O-2T (0.5) | - | B5642 (0.01) |
| Example 14 | IPTO (0.3) | O-2T (0.5) | - | B6154 (0.005) |
| Example 15 | IPTO (0.3) | O-2T (0.5) | - | LA-52 (0.0025) |
| Example 16 | IPTO (0.3) | O-2T (0.5) | - | LA-63P (0.0008) |
| Comparative Example 5 | IPTO (0.3) | - | DMBE (0.5) | Tinuvin770 (0.01) |

| | | | | |
|---|---|---|---|---|
| * Numerical values in parentheses in the table represent amounts (part(s) by mass) of components added. * Cationically polymerizable monomer : KR-470 (50 parts by mass), OXT-121 (50 parts by mass) * Photoacid generator : DPIB (0.6 parts by mass) | | | | |

**[Table 4]**

| | First Second Third | | | | | | | | |
|---|---|---|---|---|---|---|---|---|---|
| | Fabrica bility | Fabrication accuracy | | Fabrica bility | Fabrication accuracy | | Fabrica bility | Fabrication accuracy | |
| | | Average value | Surface condition | | Average value | Surface condition | | Average value | Surface condition |
| Example 6 | Good | 0.04 | A | Good | 0.04 | A | Good | 0.05 | B |
| Example 7 | Good | 0.05 | A | Good | 0.05 | A | Good | 0.05 | B |
| Example 8 | Good | 0.05 | A | Good | 0.05 | A | Good | 0.05 | B |
| Example 9 | Good | 0.04 | A | Good | 0.04 | A | Good | 0.04 | B |
| Example 10 | Good | 0.04 | A | Good | 0.04 | A | Good | 0.05 | B |
| Example 11 | Good | 0.05 | A | Good | 0.05 | B | Good | 0.05 | B |
| Example 12 | Good | 0.05 | A | Good | 0.05 | A | Good | 0.05 | B |
| Example 13 | Good | 0.05 | A | Good | 0.05 | A | Good | 0.05 | B |
| Example 14 | Good | 0.05 | A | Good | 0.05 | A | Good | 0.05 | B |
| Example 15 | Good | 0.05 | A | Good | 0.05 | B | Good | 0.05 | B |
| Example 16 | Good | 0.05 | A | Good | 0.05 | A | Good | 0.05 | B |
| Comparative Example 5 | Poor | - | - | - | - | - | - | - | - |

As shown in Examples 6 to 16 in Tables 3 and 4, when the cationically polymerizable resin compositions containing appropriate amounts of various photosensitizers, the specific thiophene compound serving as an electron-donating compound, and the basic compound in combination were used for photofabrication, fabrication proceeded to the end in all three times. On the other hand, when the cationically polymerizable resin composition containing an electron-donating compound other than a specific thiophene compound was used for photofabrication (Comparative Example 5), photofabrication did not proceed.

### <Examples 17 to 25 and Comparative Examples 6 to 9>

Cationically polymerizable resin compositions were prepared in the same manner as in Example 1, except that an activating light absorbent or a phenolic antioxidant was incorporated as described in Table 5 in addition to the photosensitizer, the electron-donating compound, and the basic compound, a first to third photofabrication were performed, and fabricability and a surface of the resulting three-dimensional photofabricated product were evaluated for each photofabrication in the same manner as in Example 1. The results are shown in Table 6.

**[Table 5]**

| | Photosen sitizer (b2) | Electron-donating compound (b3) | Basic compound (C) | Activating light absorbent (D) | | | Phenolic antioxidant (E) | |
|---|---|---|---|---|---|---|---|---|
| | IPTO | Specific thiophene compound: O-2T | Tinuvin 770 | SS703 | SS704 | SS106 | HQME | BHT |
| Example 17 | 0.3 | 0.5 | 0.01 | 0.3 | - | - | 0.05 | 0.05 |
| Example 18 | 0.3 | 0.5 | 0.01 | - | 0.3 | - | 0.05 | 0.05 |
| Example 19 | 0.3 | 0.5 | 0.01 | - | - | 0.2 | 0.05 | 0.05 |
| Example 20 | 0.3 | 0.5 | 0.01 | 0.08 | - | - | 0.005 | 0.005 |
| Example 21 | 0.3 | 0.5 | 0.01 | 1.4 | - | - | 0.05 | 0.05 |
| Example 22 | 0.3 | 0.5 | 0.01 | - | - | - | 0.05 | 0.05 |
| Example 23 | 0.3 | 0.5 | 0.01 | 0.3 | - | - | - | - |
| Example 24 | 0.01 | 0.01 | 0.0005 | 0.3 | - | - | 0.05 | 0.05 |
| Example 25 | 1 | 1.5 | 0.05 | 0.3 | - | - | 0.05 | 0.05 |
| Comparative Example 6 | 0.3 | - | 0.01 | 0.3 | - | - | 0.05 | 0.05 |
| Comparative Example 7 | 0.3 | 0.5 | - | 0.3 | - | - | 0.05 | 0.05 |
| Comparative Example 8 | 0.3 | 15 | 0.01 | 0.3 | - | - | 0.05 | 0.05 |
| Comparative Example 9 | 0.3 | 0.5 | 8 | 0.3 | - | - | 0.05 | 0.05 |

| | | | | | | | | |
|---|---|---|---|---|---|---|---|---|
| * Numerical values in parentheses in the table represent amounts (part(s) by mass) of components added. * Cationically polymerizable monomer : KR-470 (50 parts by mass), OXT-121 (50 parts by mass) * Photoacid generator : DPIB (0.6 parts by mass) | | | | | | | | |

**[Table 6]**

| | First | | | Second | | | Third | | |
|---|---|---|---|---|---|---|---|---|---|
| | Fabrica bility | Fabrication accuracy | | Fabrica bility | Fabrication accuracy | | Fabrica bility | Fabrication accuracy | |
| | | Average value | Surface condition | | Average value | Surface condition | | Average value | Surface condition |
| Example 17 | Good | 0.02 | A | Good | 0.02 | A | Good | 0. 02 | A |
| Example 18 | Good | 0.03 | A | Good | 0.03 | A | Good | 0.03 | A |
| Example 19 | Good | 0.03 | A | Good | 0.03 | A | Good | 0.03 | A |
| Example 20 | Good | 0.04 | A | Good | 0.04 | A | Good | 0.05 | B |
| Example 21 | Good | 0.03 | A | Good | 0.03 | B | Good | 0.04 | B |
| Example 22 | Good | 0.04 | A | Good | 0.04 | A | Good | 0.05 | A |
| Example 23 | Good | 0.03 | A | Good | 0.03 | A | Good | 0.04 | B |
| Example 24 | Good | 0.04 | B | Good | 0.04 | B | Good | 0.04 | B |
| Example 25 | Good | 0.04 | A | Good | 0.04 | A | Good | 0.04 | B |
| Comparative Example 6 | Poor | - | - | - | - | - | - | - | - |
| Comparative Example 7 | Good | 0.04 | A | Good | 0.04 | B | Good | 0.05 | C |
| Comparative Example 8 | Good | 0.05 | C | Poor | - | - | - | - | - |
| Comparative Example 9 | Poor | - | - | - | - | - | - | - | - |

As shown in Examples 17 to 25 in Tables 5 and 6, when the cationically polymerizable resin compositions containing appropriate amounts of the activating light absorbent or the phenolic antioxidant in addition to the photosensitizer, the specific thiophene compound, and the basic compound were used for photofabrication, fabrication proceeded to the end in all three times. The fabrication accuracy of the resulting three-dimensional photofabricated products was relatively high. On the other hand, when the cationically polymerizable resin composition that contained no specific thiophene compounds was used for photofabrication (Comparative Example 6), photofabrication did not proceed. When the cationically polymerizable resin composition that contained no basic compounds was used for photofabrication (Comparative Example 7), a decrease in fabrication accuracy was found in the resulting three-dimensional photofabricated product. When the cationically polymerizable resin compositions containing an excessive amount of the specific thiophene compound or the basic compound were used for photofabrication (Comparative Examples 8 and 9), the three-dimensional photofabricated products fell off in the middle of photofabrication.

## Claims

1. A cationically polymerizable resin composition for three-dimensional photofabrication, the composition comprising:
a cationically polymerizable monomer (A);
a photocationic polymerization initiator (B) comprising a photo acid generator (b1), a photosensitizer (b2), and an electron-donating compound (b3), the electron-donating compound being at least one thiophene compound selected from the group consisting of a monothiophene compound having a π-conjugated substituent as a substituent, a dithiophene compound, and a trithiophene compound; and
a basic compound (C),
the electron-donating compound (b3) being comprised in an amount of 0.001 to 10 parts by mass and the basic compound (C) being comprised in an amount of 0.0001 to 5.0 parts by mass relative to 100 parts by mass of the cationically polymerizable monomer (A).

2. The cationically polymerizable resin composition for three-dimensional photofabrication according to claim 1, wherein the electron-donating compound (b3) is at least one selected from a dithiophene compound or a trithiophene compound and the basic compound (C) is a hindered amine compound.

3. The cationically polymerizable resin composition for three-dimensional photofabrication according to claim 1, wherein the photosensitizer (b2) is at least one selected from the group consisting of a thioxanthone compound, an α-diketone compound, and an anthracene compound.

4. The cationically polymerizable resin composition for three-dimensional photofabrication according to claim 1, further comprising at least one selected from an activating light absorbent (D) or a phenolic antioxidant (E).

5. A method for producing a three-dimensional photofabricated product, the method comprising:
producing a stack having a shape corresponding to a shape of a three-dimensional object by digitizing and ordinating the three-dimensional object in a height direction of the three-dimensional object and generating two-dimensional shape data indicating a cross-sectional shape of the three-dimensional object at each ordinated height, based on three-dimensional shape data indicating the shape of the three-dimensional object, and sequentially forming and stacking fabricated layers each having a shape corresponding to a two-dimensional shape at each height based on the two-dimensional shape data, according to an order of the ordinating, using a vat photopolymerization device in which a predetermined position of a liquid cationically polymerizable resin composition held in a vat is irradiated with activating light being ultraviolet light or visible light to selectively cure the liquid cationically polymerizable resin composition present in the position;
the liquid cationically polymerizable resin composition being the cationically polymerizable resin composition for three-dimensional photofabrication according to any one of claims 1 to 4.

6. The method for producing a three-dimensional photofabricated product according to claim 5, wherein a dental restoration is produced as the three-dimensional photofabricated product.
